(19) Europäisches Patentamt
European Patent Office
Office européen des brevets

(11) **EP 4 343 336 A1**

(12) **EUROPEAN PATENT APPLICATION**
published in accordance with Art. 153(4) EPC

(43) Date of publication:
**27.03.2024 Bulletin 2024/13**

(21) Application number: **22802880.9**

(22) Date of filing: **18.02.2022**

(51) International Patent Classification (IPC):
*G01N 35/02* (2006.01)          *G01N 1/00* (2006.01)
*G01N 1/10* (2006.01)          *G01N 33/48* (2006.01)

(52) Cooperative Patent Classification (CPC):
**G01N 1/00; G01N 1/10; G01N 33/48; G01N 35/02**

(86) International application number:
**PCT/JP2022/006602**

(87) International publication number:
**WO 2022/244352 (24.11.2022 Gazette 2022/47)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(30) Priority: **18.05.2021 JP 2021083890**

(71) Applicant: **HITACHI HIGH-TECH CORPORATION**
**Tokyo 105-6409 (JP)**

(72) Inventors:
• **WAKUI, Akihito**
**Tokyo 105-6409 (JP)**
• **MORI, Takamichi**
**Tokyo 105-6409 (JP)**
• **TAKIZAWA, Hikaru**
**Tokyo 105-6409 (JP)**
• **IIJIMA, Masahiko**
**Tokyo 105-6409 (JP)**
• **SAGAE, Nozomi**
**Tokyo 105-6409 (JP)**

(74) Representative: **Strehl Schübel-Hopf & Partner**
**Maximilianstrasse 54**
**80538 München (DE)**

(54) **SAMPLE CONTAINER AND AUTOMATIC ANALYSIS DEVICE**

(57)     There is provided a sample container capable of dispensing a sample with a high accuracy even when a diameter of a test tube varies.

The sample container includes: a containing unit that contains a sample and is inserted into a test tube; and a flange portion that is provided at an upper end of the containing unit and mounted on an open end portion of the test tube, in which the containing unit has a tubular body portion and a cup-shaped bottom portion connected to the body portion, and in the bottom portion, an angle formed by an inner wall continuous from the body portion and a central axis has a gradient of less than 20 degrees.

[FIG. 2]

## Description

Technical Field

The present invention relates to a sample container that contains a sample to be analyzed by an automatic analyzer.

Background Art

An automatic analyzer is a device that automatically performs qualitative or quantitative analysis on a specific component contained in a sample such as blood or urine. In the automatic analyzer, when an amount of the sample is small, the sample may be contained and handled in a sample container mounted on a test tube used for collecting the sample. The sample container has a dead volume, which is an aspiration limit amount, smaller than that of the test tube, and is suitable for handling a small amount of sample. There is a plurality of types of diameters of the test tubes, and it takes time and effort to prepare the sample containers according to the types of test tubes.

PTL 1 discloses a sample container that can cope with a plurality of types of test tubes having different diameters. That is, it is disclosed that in a sample container including a containing unit that contains a sample and is inserted into a test tube and a flange portion that is provided at an upper end of the containing unit and mounted on an open end portion of the test tube, a plurality of concentric step differences corresponding to the types of test tubes are provided on a lower surface of the flange portion.

Citation List

Patent Literature

PTL 1: Design 1490791

Summary of Invention

Technical Problem

However, in PTL 1, it is insufficient to consider variation in the diameter of the test tube. The outer diameter of the test tube varies in a range of $\varphi 10$ mm to $\varphi 13$ mm with respect to a specification of $\varphi 13$ mm. Accordingly, an inner diameter of the test tube also varies in the same way. Therefore, the sample container mounted on the test tube may be position-misaligned with respect to the test tube in a horizontal direction. When the position-misalignment of the sample container with respect to the test tube is large, a sample dispensing probe for dispensing the sample from the sample container may be caught on an inner wall of the sample container, and may not be able to be lowered to a predetermined height, which may deteriorate a sample dispensing accuracy.

Accordingly, an object of the invention is to provide a sample container capable of dispensing a sample with a high accuracy even when a diameter of a test tube varies.

Solution to Problem

To achieve the object, the invention is a sample container including: a containing unit that contains a sample and is inserted into a test tube; and a flange portion that is provided at an upper end of the containing unit and mounted on an open end portion of the test tube, in which the containing unit has a tubular body portion and a cup-shaped bottom portion connected to the body portion, and in the bottom portion, an angle formed by an inner wall continuous from the body portion and a central axis has a gradient of less than 20 degrees.

Advantageous Effects of Invention

According to the invention, it is possible to provide a sample container capable of dispensing a sample with a high accuracy even when a diameter of a test tube varies.

Brief Description of Drawings

FIG. 1 is a diagram illustrating a configuration example of an automatic analyzer.
FIG. 2 is a cross-sectional view of a sample container.
FIG. 3A is a diagram illustrating a sample container mounted on a $\varphi 16$ test tube.
FIG. 3B is a diagram illustrating a sample container mounted on a $\varphi 13$ test tube.
FIG. 4 is a diagram illustrating a sample container mounted on a sample rack, the $\varphi 16$ test tube, and the $\varphi 13$ test tube.
FIG. 5 is a diagram illustrating an operation of correcting a position-misalignment of the sample container.
FIG. 6 is a diagram illustrating a shape of a bottom portion of the sample container.
FIG. 7 is a diagram illustrating a shape of the bottom portion of the sample container.
FIG. 8 is a diagram illustrating the sample container mounted on the $\varphi 16$ test tube in an inclined state.

Description of Embodiments

Hereinafter, a preferred example of an automatic analyzer according to the invention will be described with reference to the accompanying drawings. An automatic analyzer is a device that analyzes a sample such as blood or urine by using a reaction liquid obtained by reacting a reagent with the sample.

Example 1

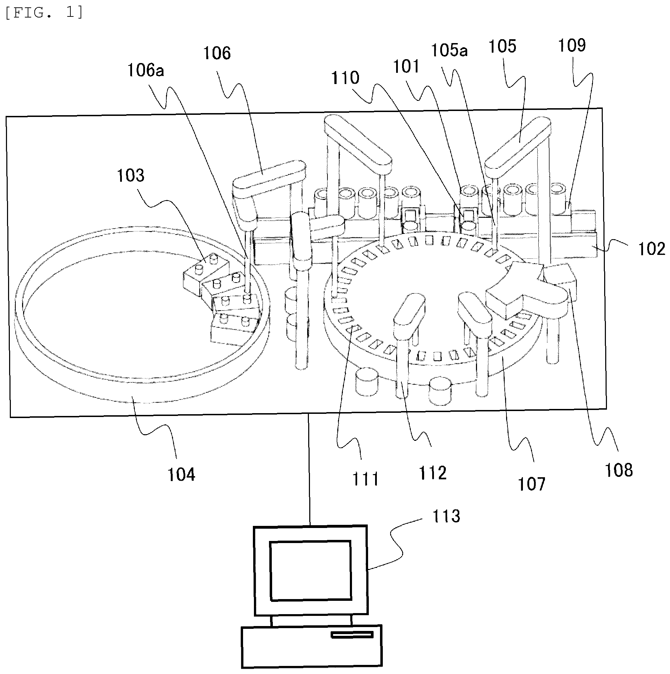

An example of an overall configuration of an automatic analyzer according to Example 1 will be described with reference to FIG. 1. The automatic analyzer includes a sample conveying unit 102, a reagent disc 104, a sample dispensing unit 105, a reagent dispensing unit 106, a reaction disc 107, a measurement unit 108, and a control unit 113. Hereinafter, each unit will be described.

The sample conveying unit 102 conveys a test tube 101 that contains the sample such as blood or urine and is placed on a sample rack 109 to a position accessible by the sample dispensing unit 105.

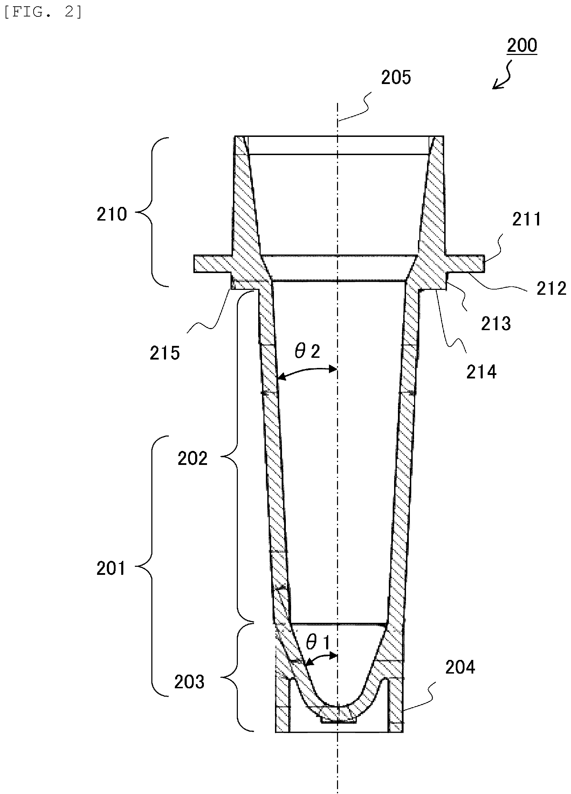

The sample dispensing unit 105 dispenses the sample from the test tube 101 conveyed by the sample conveying unit 102 to a reaction vessel 111 disposed on the reaction disc 107. A sample dispensing probe 105a included in the sample dispensing unit 105 is used for dispensing the sample. That is, the sample dispensing probe 105a is inserted into the test tube 101 to aspirate the sample, and then moves to the reaction vessel 111 to discharge the sample. When the amount of the sample is small, the sample is transferred to a sample container 200 to be described later with reference to FIG. 2. The sample container 200 is mounted on the test tube 101 and used, has a dead volume, which is an aspiration limit amount, smaller than that of the test tube 101, and therefore is suitable for handling a small amount of sample.

The reaction disc 107 keeps a plurality of reaction vessels 111 provided in a circumferential shape at a predetermined temperature range, and conveys the reaction vessel 111 into which the sample is dispensed to a position accessible by the reagent dispensing unit 106. The reagent disc 104 keeps a reagent container 103 containing a reagent to be used for analysis at a predetermined temperature range.

The reagent dispensing unit 106 dispenses the reagent from the reagent container 103 stored in the reagent disc 104 to the reaction vessel 111 into which the sample is dispensed. A reagent dispensing probe 106a provided in the reagent dispensing unit 106 is used for dispensing the reagent. That is, the reagent dispensing probe 106a is inserted into the reagent container 103 to aspirate the reagent, and then moves to the reaction vessel 111 to discharge the reagent.

The reaction vessel 111 into which the sample and the reagent are dispensed is conveyed by the reaction disc 107 to a position accessible by a stirring unit 112. The stirring unit 112 stirs the sample and the reagent in the reaction vessel 111. By the temperature keeping by the reaction disc 107 and the stirring by the stirring unit 112, a reaction between the sample and the reagent in the reaction vessel 111 is promoted, and a reaction liquid is generated. The reaction disc 107 conveys the reaction vessel 111 containing the reaction liquid to the measurement unit 108.

The measurement unit 108 measures physical characteristics of the reaction liquid contained in the reaction vessel 111, such as a light emission amount, a scattered light amount, a transmitted light amount, a current value, and a voltage value. The physical characteristics to be measured are not limited thereto. The physical characteristics measured by the measurement unit 108 are transmitted to the control unit 113.

The control unit 113 is a device that receives the physical characteristics transmitted from the measurement unit 108, outputs and stores an analysis result, controls each unit included in the automatic analyzer, and is implemented by, for example, a so-called computer.

The sample container 200 will be described with reference to FIG. 2. FIG. 2 is a cross-sectional view along a central axis 205 of the sample container 200. The sample container 200 is a rotating body around the central axis 205, and includes a containing unit 201 and a flange portion 210.

The containing unit 201 is inserted into the test tube 101 while containing the sample, and includes a tubular body portion 202 and a cup-shaped bottom portion 203 connected to the body portion 202. In the body portion 202, an inner wall may be provided with a gradient such that an inner diameter decreases from an upper end portion to a lower end portion. An angle formed by the inner wall of the body portion 202 and the central axis 205 is set to be $\theta 2$. Since a thickness of the body portion 202 is substantially uniform, when the inner wall is provided with the gradient, an outer wall thereof is also provided with the gradient.

In the bottom portion 203, an angle $\theta 1$ formed by an inner wall continuous from the body portion 202 and the central axis 205 has a gradient of less than 20 degrees, and $\theta 1 > \theta 2$. A cylindrical portion 204 having a cylindrical shape and a flat lower surface may be provided on an outer periphery of the bottom portion 203. By providing the cylindrical portion 204, the sample container 200 can stand by itself. The dead volume of the sample container 200 is determined by an inner surface shape of the bottom portion 203, and it is preferable that an inner diameter of the bottom portion is smaller to increase the dead volume. However, since it is preferable that the sample dispensing probe 105a can be inserted up to a height of about 1 mm from a bottom surface of the bottom portion 203 to aspirate a small amount of sample, the inner diameter of the bottom portion 203 at the height is larger than an outer diameter of the sample dispensing probe 105a.

The flange portion 210 is provided at an upper end of the containing unit 201, is mounted on an open end portion of the test tube 101, and has a first side surface 211, a first lower surface 212, a second side surface 213, and a second lower surface 214. Since the flange portion 210 has a step difference between the first lower surface 212 and the second lower surface 214, the sample container 200 can correspond to the test tubes 101 having different diameters.

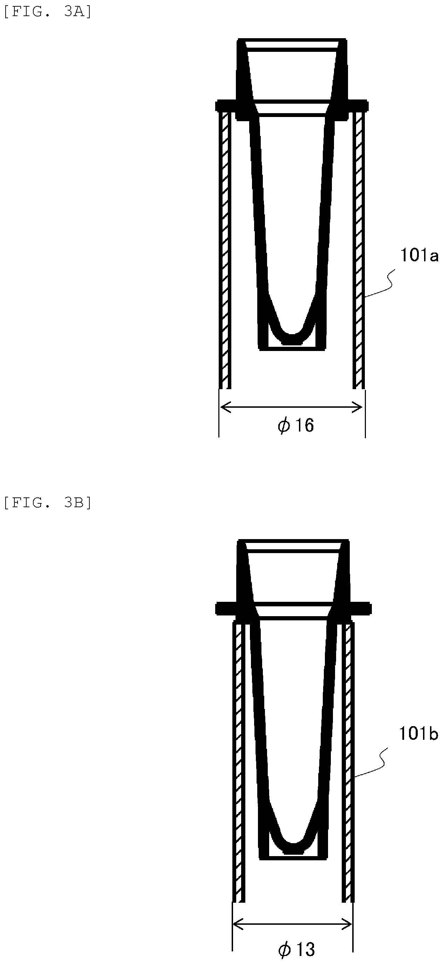

With reference to FIGS. 3A and 3B, the sample containers 200 mounted on the test tubes 101 having different diameters will be described. FIG. 3A illustrates the sample container 200 mounted on a φ16 test tube 101a having an outer diameter of 16 mm, and FIG. 3B illustrates the sample container 200 mounted on a φ13 test tube 101b having an outer diameter of 13 mm. An outer diameter of the test tube 101 is not limited to 16 mm and 13 mm.

When the sample container 200 is mounted on the φ16 test tube 101a, the first lower surface 212 is in contact with an open end portion of the φ16 test tube 101a, and a position-misalignment of the sample container 200 in a horizontal direction is limited by a distance between the second side surface 213 and an inner wall of the φ16 test tube 101a. An outer diameter of the first side surface 211 is larger than an inner diameter of the φ16 test tube 101a such that the sample container 200 does not fall into the φ16 test tube 101a.

When the sample container 200 is mounted on the φ13 test tube 101b, the second lower surface 214 is in contact with an open end portion of the φ13 test tube 101b, and the position-misalignment of the sample container 200 in the horizontal direction is limited by a distance between an outer wall of the body portion 202 and an inner wall of the φ13 test tube 101b. An outer diameter of the second side surface 213 is larger than an inner diameter of the φ13 test tube 101b such that the sample container 200 does not fall into the φ13 test tube 101b.

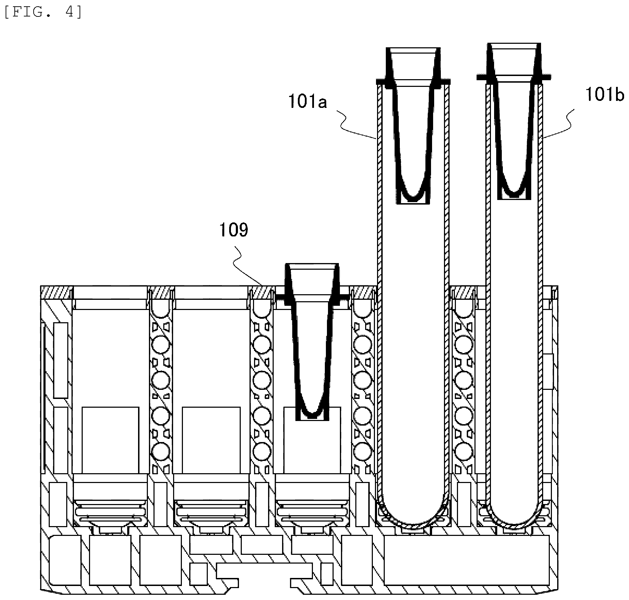

The sample container 200 may be mounted not only on the test tube 101 but also mounted on the sample rack 109 as illustrated in FIG. 4.

Since the diameter, that is, the outer diameter and the inner diameter of the test tube 101 vary depending on a manufacturer, a distance between the second side surface 213 or the outer wall of the body portion 202 and an inner wall of the test tube 101 also varies. As a result, the sample container 200 may be largely position-misaligned with respect to the test tube 101 in the horizontal direction. When the position-misalignment of the sample container 200 is large, the sample dispensing probe 105a may be caught on the inner wall of the bottom portion 203, and may not be able to be lowered to a predetermined height, which may deteriorate a sample dispensing accuracy. In Example 1, the position-misalignment of the sample container 200 in the horizontal direction is corrected by the causing the sample dispensing probe 105a descending at an appropriate speed to be in contact with the inner wall of the bottom portion 203 having an appropriate gradient. That is, since the inner wall of the bottom portion 203 is provided with a gradient of less than 20 degrees, the position-misalignment of the sample container 200 in the horizontal direction is corrected by a propulsive force in the horizontal direction generated by the sample dispensing probe 105a being in contact with the inner wall of the bottom portion 203.

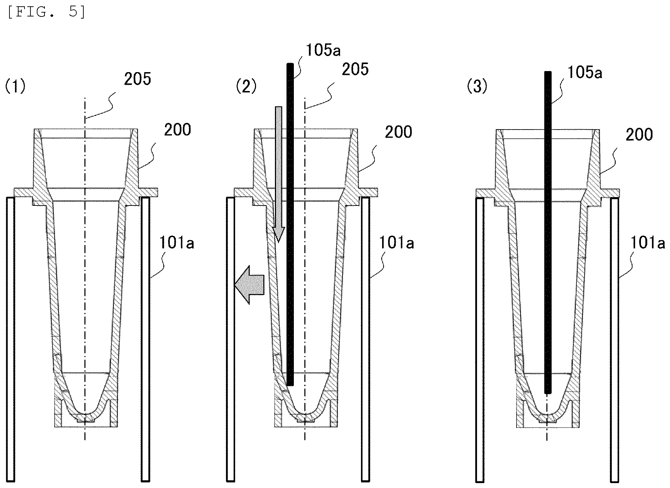

An operation of correcting the position-misalignment of the sample container 200 in the horizontal direction will be described with reference to FIG. 5. (1) of FIG. 5 illustrates the sample container 200 that is position-misaligned with respect to the φ16 test tube 101a in the horizontal direction. The position-misalignment of the sample container 200 is limited by a difference between the inner diameter of the φ16 test tube 101a and the outer diameter of the second side surface 213. Next, in (2) of FIG. 5, when the sample dispensing probe 105a is in contact with the inner wall of the bottom portion 203 in a process of descending at an appropriate speed, the propulsive force in the horizontal direction is generated. (3) of FIG. 5 illustrates the sample container 200 in which the position-misalignment with respect to the φ16 test tube 101a in the horizontal direction is corrected by the propulsive force. By correcting the position-misalignment of the sample container 200 in the horizontal direction, the sample dispensing probe 105a can be lowered to a predetermined height without being caught by the inner wall of the bottom portion 203, and the sample can be dispensed with a high accuracy.

The inventors have found from prototype experiments of the sample container 200 that it is preferable that the angle θ1 formed by the inner wall of the bottom portion 203 and the central axis 205 is less than 20 degrees. That is, when θ1 < 20 degrees, the position-misalignment of the sample container 200 can be corrected more appropriately. In FIG. 5, the operation of correcting the position-misalignment of the sample container 200 with respect to the φ16 test tube 101a is described, and position-misalignment of the sample container 200 with respect to the φ13 test tube 101b can also be corrected by the same operation as in FIG. 5.

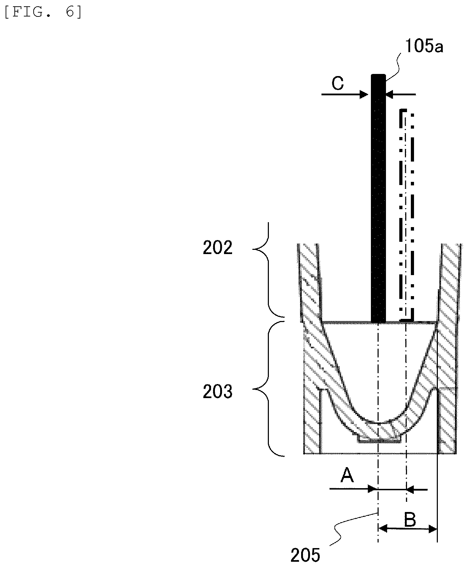

A shape of the bottom portion 203 with which the position-misalignment of the sample container 200 can be corrected will be described with reference to FIG. 6. To correct the position-misalignment of the sample container 200 by causing the sample dispensing probe 105a to be in contact with the inner wall of the bottom portion 203, it is preferable that the sample dispensing probe 105a is not in contact with the inner wall of the body portion 202, that is, the following expression is established.

$$B > A + C/2 \quad \text{...(Equation 1)}$$

Here, A is an amount of the position-misalignment of the sample container 200, B is a radius at an upper end portion of the bottom portion 203, and C is the outer diameter of the sample dispensing probe 105a.

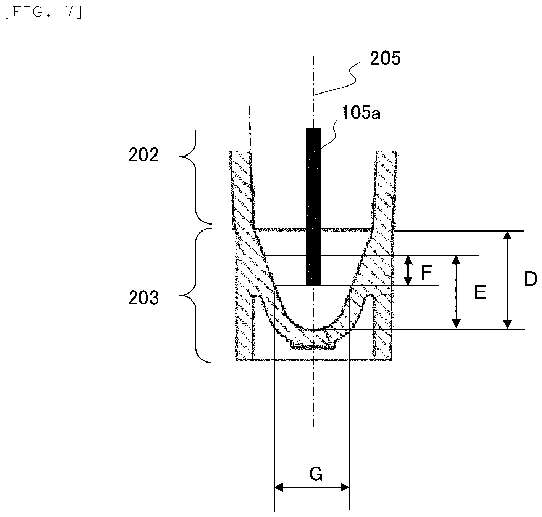

A shape of the bottom portion 203 suitable for aspirating the sample will be described with reference to FIG. 7. To allow the sample dispensing probe 105a to aspirate a small amount of sample, for example, a dead volume sample contained in the sample container 200, it is preferable that a tip of the sample dispensing probe 105a is appropriately separated from the bottom portion 203. For example, it is preferable that the tip of the sample dispensing probe 105a inserted from a liquid level height E to a depth F of the dead volume sample has a distance of Δ1 or more from the bottom surface of the bottom portion 203, that is, the following expression is established. Δ1 is, for example, 1 mm.

$$E - F \geq \Delta1 \quad ...(\text{Equation 2})$$

Further, it is preferable that the following expression is established such that the sample dispensing probe 105a inserted from the liquid level height E to the depth F is not in contact with the inner wall of the bottom portion 203.

$$G > C \quad ...(\text{Equation 3})$$

Here, G is the inner diameter of the bottom portion 203 when the liquid level height E - F.

Further, to prevent the tip of the sample dispensing probe 105a from being caught on a boundary between the body portion 202 and the bottom portion 203, it is preferable that a depth D of the bottom portion 203 is larger than the liquid level height E of the dead volume sample, that is, the following expression is established.

$$D > E \quad ...(\text{Equation 4})$$

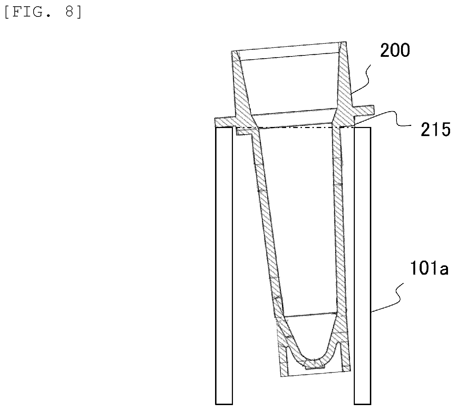

Further, as illustrated in FIG. 8, the sample container 200 may be mounted on the φ16 test tube 101a in an inclined state. When the sample container 200 is in the inclined state, the sample dispensing probe 105a cannot accurately dispense the sample.

In Example 1, in a process of conveying the test tube 101 on which the sample container 200 is mounted, the sample container 200 is in a state of being parallel to the φ16 test tube 101a, that is, the state illustrated in FIG. 3A, by vibration caused by unevenness of a conveyance path. That is, the sample container 200 makes a pendulum motion by the vibration with a contact point with the open end portion of the φ16 test tube 101a as a fulcrum, and transitions from a state inclined with respect to the φ16 test tube 101a to a state parallel thereto. In particular, the containing unit 201 inserted into the φ16 test tube 101a is suitable for the pendulum motion because an outer diameter of a lower end is smaller than an outer diameter of an upper end of the containing unit 201.

The inventors have found from the prototype experiments of the sample container 200 that it is preferable that a value obtained by dividing an outer diameter of the step difference, that is, the outer diameter of the second side surface 213 by an outer diameter of the bottom portion 203 is 1.6 or more. The inventors have also found from the prototype experiments of the sample container 200 that it is preferable that when a central axis of the test tube 101 coincides with the central axis of the sample container 200, a gap between the inner wall of the test tube 101 and an outer wall of the bottom portion 203 is 2.3 mm or more. That is, by providing an appropriate gap between the inner wall of the test tube 101 and the outer wall of the bottom portion 203, the pendulum motion can be performed in which the sample container 200 transitions from the inclined state to the parallel state. When the cylindrical portion 204 is provided on the outer periphery of the bottom portion 203, the appropriate gap is provided between an outer wall of the cylindrical portion 204 and the inner wall of the test tube 101.

In addition, since the outer diameter of the bottom portion 203 is smaller than the outer diameter of the upper end of the containing unit 201, the sample container 200 is easily inserted into the test tube 101, and air in the test tube 101 is easily released when the sample container 200 is inserted. When the air is hard to escape, there is a concern that the sample may be spilled when vibration is applied while the sample container 200 is dropping because the sample container 200 is inserted into the test tube 101 while being slowly dropped. However, such concern is resolved in the sample container 200 according to Example 1.

It is preferable that a corner 215 of the step difference of the sample container 200 is not chamfered. When the corner 215 is chamfered, the sample container 200 easily transitions from the parallel state to the inclined state due to the vibration. Therefore, by not chamfering the corner 215, the sample container 200 parallel to the test tube 101 is less likely to transition to the inclined state due to the vibration based on the unevenness of the conveyance path.

It is preferable that a height of the step difference, that is, a height from the second lower surface to the first lower surface 212 is equal to or higher than a height of the unevenness of the conveyance path. Since the height of the step difference is equal to or higher than the height of the unevenness, it is possible to prevent the transition of the sample container 200 from the parallel state to the inclined state due to the vibration. To transition the sample container 200 from the inclined state to the parallel state, it is preferable that the height of the step difference is lower. The inventors have found from the prototype experiments of the sample container that it is preferable that the height of the step difference is in a range of 0.5 mm to 2.0 mm. That is, when the height of the step difference is in the range of 0.5 mm to 2.0 mm, the sample container 200 easily transitions from the inclined state to the parallel state and hardly transitions from the parallel state to the inclined state, and therefore the sample container 200 can be maintained in the parallel state.

The example of the invention is described above. The invention is not limited to the example described above, and the components may be modified without departing from the scope of the invention. A plurality of components disclosed in the example described above may be combined as appropriate. Further, some components may be omitted from all the components described in the example described above.

Reference Signs List

101: test tube
101a: φ16 test tube
101b: φ13 test tube
102: sample conveying unit
103: reagent container
104: reagent disc
105: sample dispensing unit
105a: sample dispensing probe
106: reagent dispensing unit
106a: reagent dispensing probe
107: reaction disc
108: measurement unit
109: sample rack
110: washing tank
111: reaction vessel
112: stirring unit
113: control unit
200: sample container
201: containing unit
202: body portion
203: bottom portion
204: cylindrical portion
205: central axis
210: flange portion
211: first side surface
212: first lower surface
213: second side surface
214: second lower surface
215: corner

**Claims**

1. A sample container comprising:

a containing unit that contains a sample and is inserted into a test tube; and
a flange portion that is provided at an upper end of the containing unit and mounted on an open end portion of the test tube,
wherein the containing unit has a tubular body portion and a cup-shaped bottom portion connected to the body portion, and
wherein, in the bottom portion, an angle formed by an inner wall continuous from the body portion and a central axis has a gradient of less than 20 degrees.

2. The sample container according to claim 1,

wherein an inner diameter B at an upper end of the bottom portion satisfies B > A + C/2 when an amount of position-misalignment of the sample container with respect to the test tube in a horizontal direction is A, and an outer diameter of the sample dispensing probe used for dispensing the sample is C.

3. The sample container according to claim 1,

wherein a concentric step difference is provided on a lower surface of the flange portion, and
wherein a value obtained by dividing an outer diameter of the step difference by an outer diameter of the bottom portion is 1.6 or more, or a gap between an inner wall of the test tube and an outer wall of the bottom portion is 2.3 mm or more.

4. The sample container according to claim 3, wherein corners of the step difference are not chamfered.

5. The sample container according to claim 3, wherein a height of the step difference is equal to or higher than a height of an unevenness on an upper surface of a conveyance path to which the test tube is conveyed.

6. The sample container according to claim 1, wherein a cylindrical portion having a cylindrical shape and a flat lower surface is provided on an outer periphery of the bottom portion.

7. The sample container according to claim 1, wherein, when a sample dispensing probe used for dispensing the sample is inserted from a liquid level height E to a depth F of a dead volume sample, a tip of the sample dispensing probe is at a predetermined distance Δ1 or more from a bottom surface of the bottom portion.

8. The sample container according to claim 1, wherein a depth D of the bottom portion is D > E where E satisfies a liquid level height of a dead volume sample.

9. An automatic analyzer that analyzes the sample contained in the sample container according to claim 1, wherein, when the sample container is position-misaligned with respect to the test tube in a horizontal direction, the position-misalignment is corrected by allowing a sample dispensing probe used for dispensing the sample to be in contact with the inner wall of the bottom portion.

[FIG. 1]

[FIG. 2]

[FIG. 3A]

101a

φ 16

[FIG. 3B]

101b

φ 13

[FIG. 4]

[FIG. 5]

(1) 205
200
101a

(2) 105a
205
200
101a

(3) 105a
200
101a

[FIG. 6]

[FIG. 7]

[FIG. 8]

## INTERNATIONAL SEARCH REPORT

| International application No. |
| --- |
| **PCT/JP2022/006602** |

**A.    CLASSIFICATION OF SUBJECT MATTER**

*G01N 35/02*(2006.01)i; *G01N 1/00*(2006.01)i; *G01N 1/10*(2006.01)i; *G01N 33/48*(2006.01)i
FI:    G01N35/02 A; G01N1/00 101H; G01N1/10 N; G01N33/48 E

According to International Patent Classification (IPC) or to both national classification and IPC

**B.    FIELDS SEARCHED**

Minimum documentation searched (classification system followed by classification symbols)

G01N35/02; G01N35/04; G01N1/00; G01N1/10; G01N33/48

Documentation searched other than minimum documentation to the extent that such documents are included in the fields searched

Published examined utility model applications of Japan 1922-1996
Published unexamined utility model applications of Japan 1971-2022
Registered utility model specifications of Japan 1996-2022
Published registered utility model applications of Japan 1994-2022

Electronic data base consulted during the international search (name of data base and, where practicable, search terms used)

**C.    DOCUMENTS CONSIDERED TO BE RELEVANT**

| Category* | Citation of document, with indication, where appropriate, of the relevant passages | Relevant to claim No. |
| --- | --- | --- |
| Y | JP 3185113 U (YUOKENSCIENCE CO., LTD.) 01 August 2013 (2013-08-01) paragraphs [0002], [0013]-[0024], fig. 1-3 | 1-9 |
| Y | JP 7-260643 A (SHARP CORP) 13 October 1995 (1995-10-13) paragraphs [0015]-[0028], fig. 2-4 | 1-9 |
| A | JP 2013-142560 A (HITACHI HIGH-TECHNOLOGIES CORP) 22 July 2013 (2013-07-22) | 1-9 |
| A | WO 2016/147239 A1 (PANASONIC CORP) 22 September 2016 (2016-09-22) | 1-9 |
| A | JP 7-140137 A (TOA MEDICAL ELECTRONICS CO LTD) 02 June 1995 (1995-06-02) | 1-9 |
| A | JP 2010-078483 A (FUJIFILM CORP) 08 April 2010 (2010-04-08) | 1-9 |

☐ Further documents are listed in the continuation of Box C.        ☑ See patent family annex.

| * | Special categories of cited documents: | "T" | later document published after the international filing date or priority date and not in conflict with the application but cited to understand the principle or theory underlying the invention |
| --- | --- | --- | --- |
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
| --- | --- |
| **31 March 2022** | **19 April 2022** |

| Name and mailing address of the ISA/JP | Authorized officer |
| --- | --- |
| **Japan Patent Office (ISA/JP)** **3-4-3 Kasumigaseki, Chiyoda-ku, Tokyo 100-8915** **Japan** | |
| | Telephone No. |

Form PCT/ISA/210 (second sheet) (January 2015)

INTERNATIONAL SEARCH REPORT
Information on patent family members

International application No.

**PCT/JP2022/006602**

| Patent document cited in search report | | | Publication date (day/month/year) | Patent family member(s) | | | Publication date (day/month/year) |
|---|---|---|---|---|---|---|---|
| JP | 3185113 | U | 01 August 2013 | (Family: none) | | | |
| JP | 7-260643 | A | 13 October 1995 | (Family: none) | | | |
| JP | 2013-142560 | A | 22 July 2013 | (Family: none) | | | |
| WO | 2016/147239 | A1 | 22 September 2016 | US | 2017/0203290 | A1 | |
| JP | 7-140137 | A | 02 June 1995 | (Family: none) | | | |
| JP | 2010-078483 | A | 08 April 2010 | (Family: none) | | | |

Form PCT/ISA/210 (patent family annex) (January 2015)